(19) Europäisches Patentamt
European Patent Office
Office européen des brevets

(11) Publication number: **0 406 516 A1**

# (12) EUROPEAN PATENT APPLICATION

(21) Application number: 90101885.3

(22) Date of filing: 31.01.90

(51) Int. Cl.[5]: C07J 63/00, C07H 15/256

(30) Priority: 04.07.89 KR 899482

(43) Date of publication of application:
09.01.91 Bulletin 91/02

(84) Designated Contracting States:
DE FR IT

(71) Applicant: DONG KOOK PHARMACEUTICAL CO., LTD.
826-26, Yeoksam-dong,
Kangnam-ku, Seoul(KR)

(72) Inventor: Hwang, Byung Yong
Jugong APT 417-202, Jamsil dong,
Kangdong-ku
Seoul(KR)
Inventor: Choi, Seung Yong
Sinbanpho Apt 6-507, Banpho-dong,
Suhcho-ku
Seoul(KR)

(74) Representative: Weber, Dieter, Dr. et al
Dr. Dieter Weber und Dipl.-Phys. Klaus Seiffert Patentanwälte
Gustav-Freytag-Strasse 25 Postfach 6145
D-6200 Wiesbaden 1(DE)

## (54) A process for extracting the concentrated gymnemate from gymnema sylvestre and an equipment for using in the process.

(57) A process for extracting the concentrated gymnemate from gymnema sylvestre which is comprising the steps of sterilizing a dried gymnema sylvestre folium with heated nitrogen, extracting said gmynema sylvestre folium with buffer solution, removing fat ingredients with an organic solvent lighter than water and removing chlorophyl with an organic solvent heavier than water.

EP 0 406 516 A1

# A PROCESS FOR EXTRACTING THE CONCENTRATED GYMNEMATE FROM GYMNEMA SYLVESTRE AND AN EQUIPMENT FOR USING IN THE PROCESS

## Technical Field

This invention relates to a process for extruding of gymnemate from gymnema sylvestre and an equipment for using in the process.

## Background of the Invention

Gymnema sylvestre distributed broadly in the tropics and the subtropics of Africa, India and China, and it is the Hindustani having the meaning of "Breaking the Sugar", and well-knowns as "GUR-MA". Since old times, the extract of gymnema sylvestre is known to have therapeutic effect of gastroenteric disorder, diabetes and cough.

Also, it has the character of breaking the sweetness tentatively, and has antibiotic property, and is greatly reduced the blood sugar level [Arogya-J. Health Sc., VII, 38, 1981].

Therefore, after chewing the leaf of gymnema sylvestre, we cannot taste the sweetness of the sweet material such as sodium saccharin, sodium cyclandate for some hours, and then if drinking a sweet tea, we can feel only the fragrance of the tea without the sweet taste.

This character of gymnema sylvestre folium is due to the action of its main ingredient, gymnemic acid.

Gymnemic acid is a saponin composed of triterpenoid aglycon and a gluconic acid. It is used to reduce the blood sugar level because it is known to excellently inhibit the intestinal absorption of glucose.

Hereupon, in order to extract the concentrated gymnemate from gymnema sylvestre folium, the present inventors have been investigated the chemical and physical properties of gymnemic acid.

As a result, it was found out the facts that the gymnemic acid is hydrogenated under the acidic or alkaline condition and so lose the properties of it easily.

Therefore, we get to this invention by developing a new method for extracting and concentrating the gymnemic acid under the buffer condition.

## Summary of the Invention

It is an object of the present invention to provide a process and an equipment for extracting the concentrated gymnemate having same activity as gymnemic acid from gymnema sylvestre folium.

## Detailed Description of the Invention

The present invention is directed to a process for extracting a : concentrated gymnemate from the gymnema sylvestre folium comprising the steps of

a) sterilizing a dried gymnema sylvestre folium with nitrogen gas heated at 120 °C,

b) extracting said gymnema sylvestre folium with buffer solution of phosphoric alkaline salt of pH 7 heated at 60 °C,

c) removing fat ingredients with an organic solvent lighter than water, and

d) removing the chlorophyl with an organic solvent heavier than water.

Also, this invention relates to an equipment for extracting the concentrated gymnemate salt according to the said process, as shown in Fig. 1.

Fig. 1 is shown a system for extracting the concentrated gymnemate from the leaf of gymnema sylvestre.

The equipment in accordance with this invention composing of an extracting column (1) filled the gymnema sylvestre folium, a pressure control valve (2), a sheat exchanger (3), a first solvent extracting equipment (4), a second solvent extracting equipment (5), a buffer solution container (6) and a pressing pump (7) as a cycling system in the order, a nitrogen pouring tube (8) attached a heat exchanger (3′) connected to the extracting column (1), and a concentrater (9) connected between the second solvent extracting equipment (5) and the buffer solution container (6).

And, herein the extracting column (1) has numerous plates (11) installed in it layer upon layer.

The present invention is further illustrated as follows.

In this invention, gymnemic acids extracted by the above equipment has the following formula (I), so they may be constituted 4 types by functional groups $R_1$, $R_2$ and $R_3$.

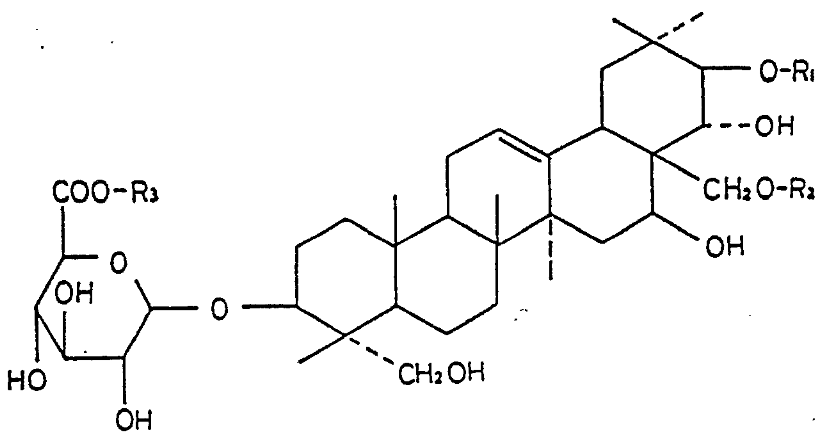

(I)

wherein $R_1$, $R_2$ and $R_3$ may be independently tigroyl, 2-methyl butyroyl or acetyl group or hydrogen atom.

Herein, the 4 types of gymnemic acids are named gymnemic acid (A), (B), (C) and (D) according to the functional group $R_1$, $R_2$ and $R_3$ as follows.

| Types | $R_1$ | $R_2$ | $R_3$ |
|---|---|---|---|
| (A) | $-CO-C(CH_3)=CH-CH_3$ | $-CO-CH_3$ | H |
| (B) | $-CO-CH(CH_3)-CH_2-CH_3$ | $-CO-CH_3$ | H |
| (C) | $-CO-CH(CH_3)-CH_2-CH_3$ | H | H |
| (D) | $-CO-C(CH_3)=CH-CH_3$ | H | H |

These gymnemic acids are easily hydrolyzed under the acidic or alkaline condition, and as the result, they lose the activity of glycolysis.

That is to say, gymnemagenin represented by following structure formula (III) and glucoronic acid represented by following structure formula (IV) are gained by the acid hydrolysis of gymnemic acid of the above formula (I), wherein the gymnemic acid is changed to prosaponin of the following structure formula (II) by the alkaline hydrolysis, and it is changed to the gymnemagenin and the glucuronic acid after the acidic hydrolysis as the following reaction,

Gymnemic acid ( I )

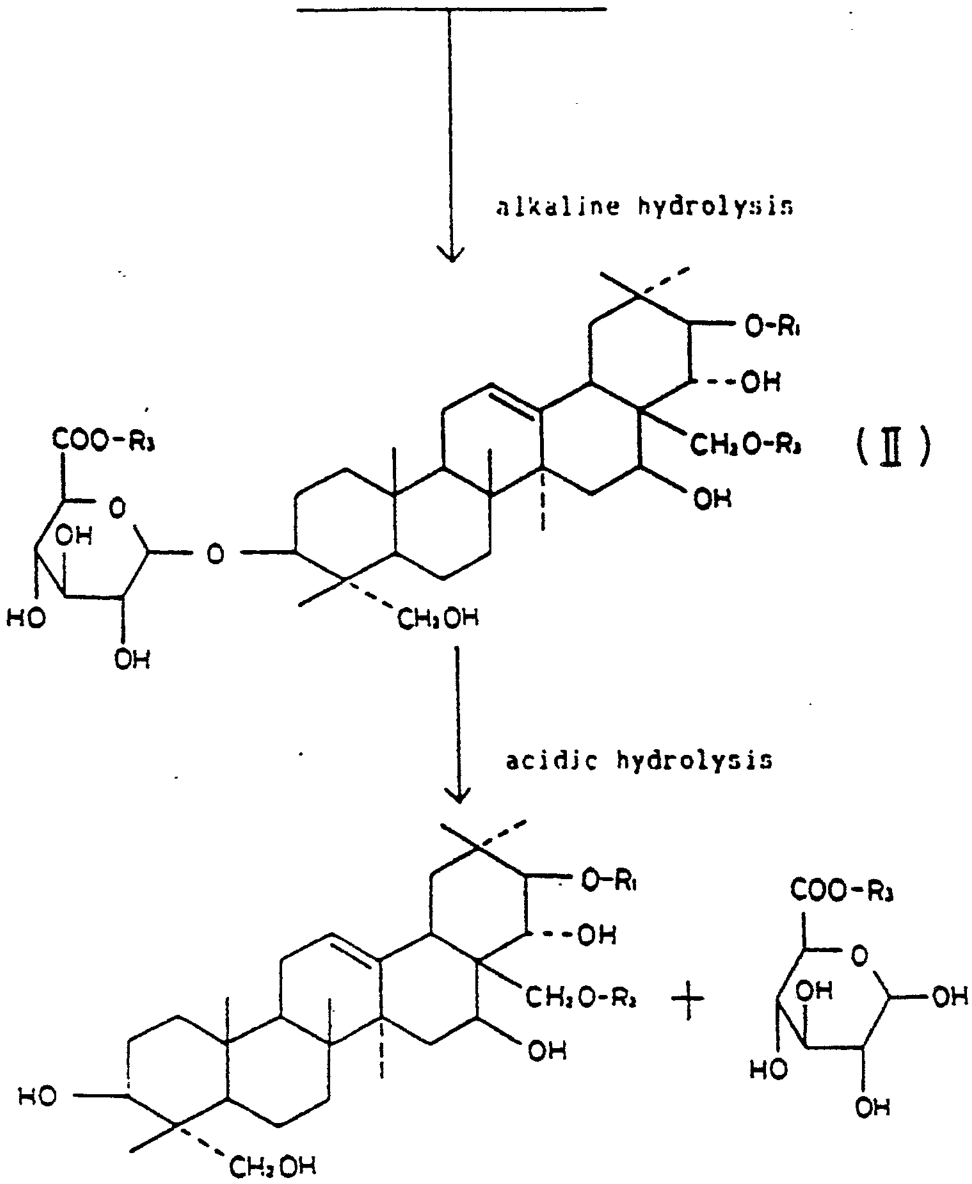


( II )

(III) (IV)

wherein, in the formula (II), $R_1$ and $R_2$ are independently hydrogen atom, $R_3$ is selected from hydrogen atom and alkaline metal, and in the formula (III) and (IV), $R_1$, $R_2$ and $R_3$ are respectively hydrogen atom.

But, when both $R_1$ and $R_2$ are acylated in the formula (I) fo gymnemic acid, that is, it is the structure of the above type (A) or (B), the gymnemic acid (A) and (B) of 0.5 mole have the activity to glycolyze 0.4 mole of sucrose.

And, when $R_1$ or $R_2$ is acylated, the gymnemic acid (C) and (D) of 1 mole may be glycolyzed 0.4 mole of sucrose.

Namely, if the number of acyl group is reduced, the activity of glycolysis is reduced, and if increased, the activity of glycolysis is also increased.

Therefore, if the gymnemic acids are hydrolyzed in the extraction step to prosaponin of the formula (II) or gymnemagenin of the formula (III), these compounds do not show activity of glycolysis because of having no acyl group.

So, in this invention, the buffer solution of phosphorous alkaline salt is used in order to extract gymnemic acid without the hydrolysis.

Process for extracting and separating the concentrated gymnemate salt is further illustrated with the Fig. 1 as follows.

First of all, the dried gymnema sylvestre foliums are tightly filled on the plates (11) in the extracting column (1), and nitrogen gas heated at 120 °C in the heat exchanger (3′) is passed from the lower part of the extracting column (1) to the upper part during about 5 min to sterilize each kind of microbiology existing in the foliums.

Then, cooled nitrogen gas is passed through the nitrogen pouring tube (8) to the lower part of the column (1) during about 5 min to cool the dried foliums.

The neutral buffer solution of phosphorous alkaline, is flowed into the lower part of the column (1) by the pump (7), which heated at 60 °C in the buffer solution container (6). At this time, the reached time of the buffer solution to the upper part in the column (1) is required about 30 min.

An extracted substance passed through the extracting column (1) passes the heat exchanger (3), and is transferred into the first solvent extracting equipment (4), wherein the fat ingredients in the extracted substance are removed.

Then, the water solution layer (4′) separated in this step is only passed through the second solvent extracting equipment (5) to remove the chlorophyl ingredient, and the water solution layer (5′) is only transfered to the buffer solution container (6).

The transfered extraction substance into the container (6) in accordance with the above step is repeatedly heated at 60 °C to circuit the above process during about 18 h.

According to this invention, in the first solvent extracting equipment (4), organic solvent (4″) is generally used hydrocarbon solvents which are lighter than water and simultaneously have good extracting effect for lipids, particularly N-hexane, heptane, petroleum ether and so on may be used desirably.

And, in the second solvent extracting equipment (5), the solvent (5″) is usually used as halogenized hydrocarbon solvents which are heavier than water and simultaneously have good extracting for the plant chlorophyl. If the solvent is chloroform, dichloroethane or carbon tetrachloride, we may be gained the desirable effect.

The gymnema sylvestre extracted by the above process is a concentration containing highly pure potassium gymnemic salt. It is suitable for manufacturing medicine because of its activity as the same with gymnemic acid.

The gymnemate concentrated in accordance with this invention is dissolved in water and controlled to pH 2 by 10 % HCl, and recrystalized in solvent which ethyl acetate is mixed with acetone to the ratio of 1 : 1 in order to the purification of it.

This invention is detailed by examples as follows.

**Example 1**

Gymnema sylvestre folium of 520 g dried and subdivided at a shade spot was tightly filled on the plates in the extraction column. The nitrogen gas heated at 120 °C by the heat changer was penetrated from the lower part to the upper part of extracting column during about 5 min.

Then, the temperature of nitrogen gas was lowered to 20 °C by the heat exchanger, and penetrated as the same with the above method into the extracting column to lower the temperature of the heated column and the dried folium.

After controlling the temperature of phosphorous alkaline buffer solution in the buffer solution container to 60 °C it was provided into the column from the lower part to the upper part by the pump.

Wherein, the valve for controlling pressure was controlled to have the passing time of the buffer solution of 30 min in the column.

Then, the buffer passed the column was cooled to 20 °C by the heat exchanger, and gone through the first solution equipment which contained the mixture 8 l of the n-heptane and ethylacetate (v/v = 1 : 1).

The solution separated at the lower part in the first solution extracting equipment was transfered into the second solution extracting equipment which contained the mixture of carbon tetrachloride and iso-propanol (v/v = 2 : 1), wherein the solution separated at the upper part was only removed into the phosphorous alkaline buffer solution container to heat at 60 °C, and the buffer solution was repeatedly provided into the column.

The above cycling process was repeated for 18 h. As the result, the buffer solution was passed the column about 36 cycles, and all the effective ingredient was extracted almost.

At this time, the buffer solution of final cycles was passed a filter and transfered into the concentrater, wherein the solution was concentrated with stirring at the temperature of it 95 °C.

The contraction was finished at the point that water is evaporated almost. Before the extract is concreted, it was transfered into the packing container and taken custody. The weight of this concentrated product was 120 g.

The other hand, the organic solvent used in the first solution extracting equipment was recovered by distillation under the reduced pressure and used in the next cycle. Then the residue was transfered and collected into the packing container before concreted. The weight of residue was 7.3 g.

Also, the solvent of the second solution extracting equipment was recovered under the condition of reduced pressure and used in the next cycle. Then the residue was transfered and collected into the packing container before concreted. The weight of residue was 6.8 g.

At this time, the residues according to the recovery of the extracting equipments were almost fat ingredient or chlorophyl, but the concentrate product from the water solution of if was mainly potassium gymnemate or an effective ingredient suitable for medicine formulation.

**Example 2**

Gymnema sylvestre folium of 550 g dried and subdivided was tightly filled on the plate in the extracting column. The nitrogen gas heated at 120 °C by the heat exchanger was penetrated from the lower part to the upper part of the column during about 5 min to sterilize the infected noxious microorganism.

Then, the temperature of nitrogen gas was lowed to 20 °C by the heat exchanger, and penetrated as the same with the above into the column to lower the temperature of the heated column and the dried folium.

After lowering the temperature of the buffer solution in container to 60 °C it was compulsorily transfered into the column from the lower part by the pump. Wherein, the valve for controlling pressure was adjusted to have the passing time of the buffer solution of 30 min in the column.

Then, the buffer passed the column was cooled to 20 °C by the heat exchanger, and sprayed into the first solution extracting equipment which contained N-hexane of 8 l, wherein the fat ingredient including in the extracted solution was dissolved.

So, the extracted solution of phosphorous alkaline salt separated at the lower part in the equipment was sprayed, to the lower part, into the second solution extracting equipment which contained chloroform, for dissolving the chlorophyl, wherein the extracted solution separated to the upper part was transfered into the container for the phosphorous alkaline salt buffer solution. And the buffer solution heated to 60 °C in the container was repeatedly provided into the column.

The above cycling process was repeatedly continuous. As the result, the process was progressed during about 18 h. Then, the buffer solution as extracted solution of the final cycle ws transfered into the concentrater, and it was entirely concentrated under a strong whirlwind and the condition of the atmosphere.

The resulted extracting solution appeared Rf value of 9 spots such as 0.62, 0.49, 0.43, 0.35, 0.31, 0.29, 0.17, 0.14 and 0.10 by developing and coloring in the solvent consisted of chloroform/acetic acid/methanol of 1 : 1 : 1 by the ratio of volume.

The main ingredient of the extracted product was potassium gymnemate, and the product was dissolved in 10 % water, and adjusted to pH 2 by hydrochloride to deposit the gymnemic acid. Then it was stirred at 10 °C during 30 min and dried to gain crude gymnemic acid of 22 g.

The gained crude cymnemic was recrystallized in solvent 100 ml mixed ethyl acetate and acetone (1 : 1 by the ratio of volume) to gain white microcrystal having melting point 199 ~ 201 °C, and it was appeared Rf valve of 2 spots by developing in the solvent consisted of butylformiate/methyl ethyl-ketone/formic acid-water of 5 : 3 : 1 : 1 by the ratio of volume.

**Claims**

1. A process for extracting a concentrated gymnemate from the gymnema sylvestre foliums comprising the steps of

a) sterilizing a dried gymnema sylvestre folium with nitrogen gas heated at 120 °C,

b) extracting said gymnema sylvestre folium with buffer solution of phosphorous alkaline salt of pH 7 heated at 60 °C,

c) removing fat ingredients with an organic solvent lighter than water, and

d) removing chlorophyl with an organic solvent heavier than water.

2. The process as recited in claim 1, wherein said organic solvent in the c) step is selected from the group

consisting of N-hexane, heptane and petrolium ether.

3. The process as recited in claim 1, wherein said organic solvent in the d) step is selected from the group consisting of chloroform, ethylene dichloride and carbon tetrachloride.

4. The process as recited in claim 1, wherein said concentrated gymnemate is additionally dissolved in water and adjusted to pH 2 by hydrochloride, and it is recrystallized in solvent mixed ethyl acetate and acetone of 1 : 1 by the ratio of volume.

5. An equipment for extracting a concentrated gymnemate from the gymnema sylvestre folium composing of an extracting column(1) filled the gymnema sylvestre foliums, a pressure control valve (2), a heat exchanger (3), a first solvent extracting equipment (4), a second solvent extracting equipment (5), a buffer solution container (6) and a pressing pump (7) as a cycling system in the order, a nitrogen pouring tube (8) attached a heat exchanger (3′) connected to the extracting column (1), and a concentrater (9) connected between the sec.nd solvent extracting equipment (5) and the buffer solution container (6).

6. The equipment as recited in claim 5, wherein said extracting column (1) has numerous plastes (11) installed in it layer upon layer.

FIG. 1

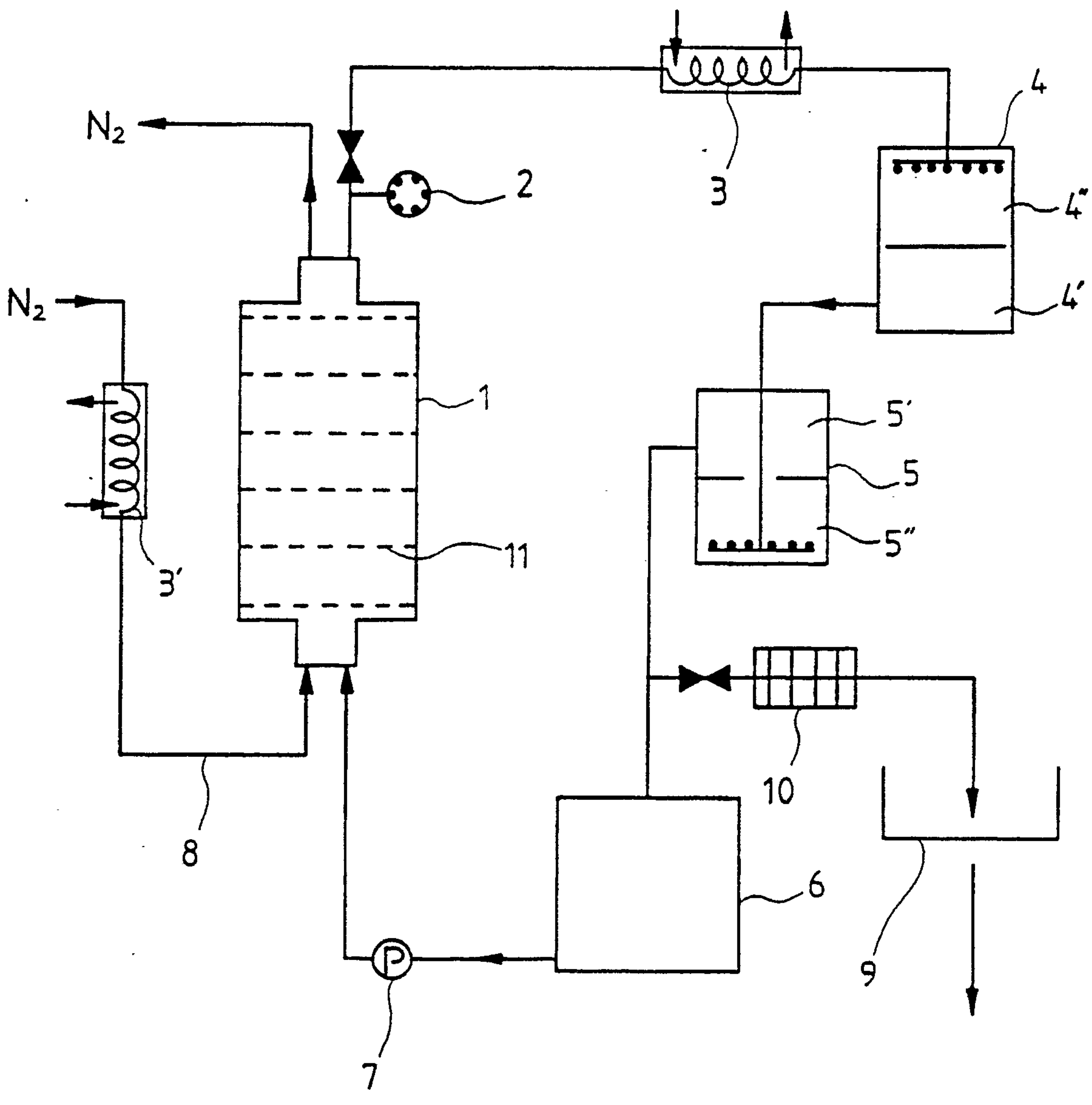

European Patent Office

# EUROPEAN SEARCH REPORT

Application number

EP 90101885.3

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl.5) |
| A | JOURNAL OF AGRICULTURAL AND FOOD CHEMISTRY, vol. 21, 1973 G.P. DATEO JR. et al. "Gymnemic Acid, the Antisaccharine Principle of Gymnema Sylvestre. Studies on the Isolation and Heterogeneity of Gymnemic Acid A1" pages 899-903 * Pages 899-901 * -- | 1-6 | C 07 J 63/00<br>C 07 H 15/256 |
| A | TETRAHEDRON LETTERS, vol. 30, no. 12, 1989 M. MAEDA et al. "Studies on taste modifiers II. Purification and structure determination of gymnemic acids, antisweet active principle from Gymnema sylvestre leaves" pages 1547-1550 * Pages 1547-1549 * -- | 1-4 | |
| | | | TECHNICAL FIELDS SEARCHED (Int Cl.5) |
| A | JOURNAL INDIAN CHEMICAL SOCIETY, vol. 35, 1958 H.N. KHASTIGIR et al. "Chemical investigation on the leaves of gymnema sylvestre R.Br." pages 650-652 * Pages 651,652 * -- | 1-4 | C 07 J 63/00<br>C 07 H 15/00 |
| A | HELVETICA CHIMICA ACTA, vol. 50, 1967 W. STÖCKLIN et al. "Gymnenasäure, das antisacaccharine Prinzip von Gymnema sylvestre R.BR. Isolierungen und Identifizierungen" pages 474-490 * Pages 477,478,484,485 * ---- | 1-4 | |

The present search report has been drawn up for all claims

| Place of search | Date of completion of the search | Examiner |
|---|---|---|
| VIENNA | 13-09-1990 | PETROUSEK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document